Europäisches Patentamt

European Patent Office

Office européen des brevets

(19)

(11) Publication number: **0 330 140**
**A2**

(12) **EUROPEAN PATENT APPLICATION**

(21) Application number: **89102991.0**

(22) Date of filing: **21.02.89**

(51) Int. Cl.⁴: **A61K 7/32**

(30) Priority: **22.02.88 US 158437**

(43) Date of publication of application:
**30.08.89 Bulletin 89/35**

(84) Designated Contracting States:
**BE CH DE ES FR GB GR IT LI NL SE**

(71) Applicant: **Bristol-Myers Company**
**345 Park Avenue**
**New York New York 10154(US)**

(72) Inventor: **Alonso, Richard J.**
**11 Dorrien Road**
**Livingston, New Jersey 07039(US)**
Inventor: **Tencza, Thomas M.**
**31 Wagner Avenue**
**Wallington, N.J. 07057(US)**
Inventor: **Grabois, Daniel M.**
**48 Dolores Drive**
**Edison, New Jersey 08817(US)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) **Hybrid antiperspirant compositions.**

(57) Antiperspirant suspension compositions containing a volatile silicone component, a non-volatile silicone component, an active antiperspirant ingredient and water are disclosed.

EP 0 330 140 A2

## HYBRID ANTIPERSPIRANT COMPOSITIONS

### Field of the Invention

This invention relates to the field of lipophilic antiperspirant compositions containing dimethicone, a cyclomethicone oil, an aluminum or aluminum/zirconium salt and from about 15% to about 30% by weight of water.

### Background of the Invention

The use of various antiperspirant compounds (including compounds similar to those used as constituents in the compositions of the present invention), in anhydrous suspensions is known in the art.

Generally speaking anhydrous suspensions are utilized extensively as so called "dry roll-on" antiperspirant compositions. Such anhydrous suspensions have relatively high cost and exhibit an "oily" feeling which, while not necessarily undesirable, does not present the same elegant feeling as do the hybrid compositions of the present invention.

Exemplary of various prior art teachings in this area of which the applicants are aware, are the following:

U.S. Patent 4,552,753 to Elm and Lowrey, which describes an anhydrous suspension roll-on similar to the presently claimed compositions. The compositions of Elm and Lowrey do not however contain water.

U.S. Patent 4,122,029 to Gee and Keil, which describes stable emulsions of a polar liquid in a non-polar base liquid. This patent requires the addition of 1-10% polydiorganosiloxanepolyoxyalkylene copolymer.

U.S. Patent 4,065,564 to Miles and Netzbandt, which describes a product with 40-85% ethanol in a base that is essentially anhydrous. In contrast to Miles and Netzbandt the compositions of the present invention do not contain alcohol and contain 20-25% water.

U.S. Patent 4,053,581 to Pader et al., which describes a composition which is similar to that described in U.S. Patent 4,065,564.

U.K. Application No. 2,155,337 to Deckner, which describes a product with 20% alcohol.

Netherlands Application NL 81/284, which describes an anhydrous aerosol product.

Belgium Patent BE 879,716 to Woodward, which describes water-in-oil emulsions.

German Offen DE 3,045,083 to Thimineur et al., which describes a vehicle for use in silicone/water emulsions.

Canadian Patent 1,076,030 to Wega, which describes a water-in-oil emulsion.

German Offen DE 2,850,488 to Hughett, which describes a composition having a water-in-oil emulsion.

U.K. Patent Application G.B. 2,018,590 to Elm et al., describes a composition similar to the composition of the present invention. However, as the Elm et al., compositions do not contain water, they are distinctly different.

None of the prior art references of which the applicant is aware teaches all the components of the hybrid compositions of the present invention which have been found to possess a unique combination of characteristics which render them particularly well suited for use as antiperspirant roll-on compositions.

### Summary of the Invention

The present invention is directed to lipophilic antiperspirant compositions containing dimethicone, cyclomethicone oils, aluminum or aluminum zirconium salts and from about 15% to about 30%, and preferably from about 20% to about 25%, by weight of water.

The compositions disclosed are uniquely suited for use as antiperspirant roll-on compositions, particularly as lower cost replacements for conventionally employed "dry" roll-ons.

### Description of the Invention

It has been found that lipophilic antiperspirant compositions can be prepared having substantial quantities of water in place of the cyclomethicone compounds conventionally employed in such "dry" roll-on compositions. The lipophilic antiperspirant compositions of the present invention possess excellent quick drying properties, possess a more elegant feel and are substantially less costly.

More particularly, the benefits to be achieved by the hybrid antiperspirant compositions of the present invention are as follows:

1. They are significantly lower in cost than anhydrous suspensions.

2. The hybrid roll-on compositions are quick drying and perhaps only slightly slower drying than an anhydrous suspension.

3. The hybrid compositions impart a "wetness" signal, allowing the user to be aware that the product is being applied. It therefore helps prevent the over application of product.

4. The hybrid compositions are less oily in feeling than anhydrous suspensions.

5. The hybrid compositions possess excellent organoleptic properties and have the elegant smoothness of an oil-in-water emulsion, without feeling watery.

It has been found that the hybrid compositions of the present invention are not electrical conductors and it is, therefore surmised that the water in the product is colloidally dispersed. It has also been determined that most of the active ingredient is soluble in the water phase and that the hybrid contains a very small (if any) amount of suspended solid antiperspirant active ingredient.

The key ingredients of the compositions of the present invention are the active antiperspirant ingredient, the volatile silicone(s), the non-volatile silicone(s) and water. Typical formulation(s) for the hybrid roll-on compositions disclosed herein are set-out in Table 1.

The addition of water to prior art compositions would result in separation of the composition taught therein into two layers.

Water in the present composition permits a reduction in cost and provides stable compositions which do not gel and are readily dispersible by ordinary shaking. The terms "stable" or "stability" as used herein mean the composition does not separate or, if it does separate, it is readily reconstituted by gentle shaking and does not exhibit gelation within three months after being stored at an elevated temperature of about 104°F. Water further provides a wetness feel (signal) without being wet. This permits the consumer to control usage. When the application provides a wet feel, the consumer knows that a sufficient quantity has been applied. Water also eliminates the oily feel provided by over application when using anhydrous systems and in addition, provides a composition which gives a smooth feel to the touch when wet.

Water also solubilizes the active antiperspirant and it is believed that the solubilized active antiperspirant may be more readily available to prevent perspiration than when the active antiperspirant is in a volatile media, where it may have to first be solubilized in perspiration.

## TABLE 1

### General Formula - Hybrid Roll-On Compositions

| Ingredient | Weight % | Purpose |
|---|---|---|
| *Dimethyldioctadecyl ammonium hectorite | 0.5 - 2.0, preferably 1.0 - 1.5 | Viscosity control, suspending aid |
| *Lower alky carbonate, preferably propylene carbonate | 0.0 - 0.8, preferably 0.5 | Helps swell and expand hectorite |
| *Dimethicone | 5.0 - 12.0, preferably 10.0 | Non-volatile lubricant prevents whitening on skin and aids in prevention of gelation |
| **Colloidal silicon dioxide | 0.0 - 0.5 | Suspending aid |
| **Polyethylene powder | 3.0-7.0 and preferably 5.0 | Lends creaminess to formula, auxiliary suspending aid |
| **Butylated hydroxytoluene | 0.00 - 0.05 | Antioxidant, perfume stabilizer |
| **Ethylene diamine tetraacetic acid (Disodium salt) | 0.00 - 0.1 | Chelating agent, perfume stabilizer |
| *Nonionic emulsifier (lipophilic nonionic emulsifier, preferably HLB 3-9, more preferably 6-8 most preferably 7) | 0.05 - 1.0 | An aid in dispersion of water |
| *Glycols:Propylene-glycol, 2-ethyl-1,3-hexanediol, glycerin, dipropylene glycol and most preferably 2-ethyl-1,3-hexanediol | 0.05 - 5.0 preferably 0.8 | Cosolvent, as aid in dispersion of water and viscosity control |

4

| **Perfumery materials | 0.0 - 0.5 | Fragrance |
| *Water | 15 - 30% preferably 20 - 25. | Imparts wetness signal |
| *Aluminum or aluminum zirconium antiperspirant salt | 16 - 26 preferably 18 - 24 | Active ingredient |
| *Cyclomethicone (pentamer and tetramer) | qs to 100 | Volatile silicone |

*The ingredients are essential to obtaining the desired product of the present invention.

**These ingredients produce a more desirable product when added.

While the glycols such as propylene glycol, 2-ethyl-1, 3 hexanediol, and the like may be employed at about 0.1 to 5.0 percent by weight, it is preferred to employ such ingredients at levels of about 0.8 to about 2.0 percent by weight.

Generally speaking, the concentrations of the various ingredients can be varied to achieve a greater or lesser effect, as may be deemed desirable, and will be within the ability of one skilled in the art to determine without undue experimentation. Therefore, the precise amount of each ingredient within the scope of the aforementioned typical ranges will not be elaborated on further here.

It has however been found that the amount of water present must be within a certain well defined range in order to achieve the degree of "wetness" and the particular feel which has been deemed desirable at the particular levels of concentration of the other recited ingredients present.

More specifically, the water component of the formulations of the composition of the instant invention must be present in an amount from about 15 to about 30 weight percent and, more preferably, will be present in an amount from about 20 to about 25 weight percent.

The aluminum or aluminum zironium salt component of the aforementioned typical formulation can be any aluminum salt or aluminum zironium salt which has antiperspirant activity. Preferably the aluminum salt used will be selected from aluminum chlorohydrate, aluminum sesquichlorohydrate and aluminum dichlorohydrate. Aluminum chlorohydrate has been found to be particularly advantageous.

Preferably the aluminum zirconium salt used will be selected from aluminum zirconium tetrachlorohydrex-Gly, pentachlorohydrex-Gly and octachlorohydrex-Gly. Aluminum zirconium tetrachlorohydrex-Gly has been found to be particularly useful.

Other useful aluminum salts are identified in the OTC drug review regulations set forth in Federal Register Vol. 47, No. 162 at pp 36492-36505

An effective amount of an antiperspirant is one which will produce a 20% reduction of perspiration according to the OTC drug review regulations.

In addition, various emulsifiers and cosolvents, such as Trideceth-3, and 2-ethyl-1,3-hexanediol, may be added to facilitate dispersion and improve stability. These are materials that are known to the cosmetic chemist, and are used to modify viscosity and to improve stability. Nonionic emulsifiers, cosolvents such as glycols, and alcohols and other such emulsifiers and cosolvents known in the art that are not reactive with the active salt may be used.

The advantages of the hybrid roll on compositions of the present invention are that they:

1. are dry
2. leave a signal
3. impart good organoleptics
4. are stable
5. are cost effective

To achieve advantages 1 through 3, and 5 it is necessary to replace part of the formula amount of cyclomethicone with water (15 to 25% replacement). To achieve item 4 (stability, as defined previously), it is necessary to add an emulsifier (e.g. trideceth -3) and an antigellant/viscosity modifier (e.g. 2-ethyl-1,3 hexanediol). In addition, it is necessary to manufacture the product by a method whereby sufficient homogenization is accomplished. A Waring Blender was used for laboratory batches, while a Manton-Gaulin homogenizer was used for production and pilot plant scale-up.

In the aqueous vehicle of the present invention, certain salts, particularly ZAG, tend to gel upon aging. ACH, on the other hand, does not tend to cause the aqueous vehicle to gel upon aging. To avoid gelation and improve stability, additives such as 2-ethyl 1,3 hexane diol, dipropylene glycol and propylene glycol (antigellants/viscosity modifiers); trideceth-3, steareth-2 (lipophilic emulsifiers) are added to help disperse the water into the oily base. Low HLB emulsifiers are used to prevent converting the product into a less organoleptically acceptable O/W emulsion.

While the additives help prevent gelation with ZAG, for example, they are not essential to systems with ACH.

While the invention has been described above, the claims of the present invention will be better understood by recourse to the following examples:

## EXAMPLES

## GENERAL COMMENTS

The following preparation steps should be employed to successfully carry out the present invention unless otherwise indicated.

### Part I (Gel preparation)

1. Disperse Bentone 38 in 13.0% of Cyclomethicone D-5 pentamer and Lightnin' mix for 15 minutes
2. Add propylene carbonate and Lightnin' mix for 15 minutes
3. Pass gel through Manton-Gaulin homogenizer set at 5000 psig (plus or minus 2000 psig).

### Part II (Addition of remainder of base ingredients)

1. Into a stainless steel container, add the remaining amount of cyclomethicone, D-5 pentamer, Cyclomethicone D-4 tetramer, dimethicone, butylated hydroxytoluene and dipropylene glycol, colloidal silicon dioxide, polyethylene B-6 and fragrance, and the Part I gel preparation. Lightnin' mix until uniform,
2. Pass the Part II (1) through a Manton-Gaulin at 5000 psig (plus or minus 2000 psig), and recirculate until the outlet temperature is 110° F.

### Part III (Addition of remaining ingredients)

1. Add ZAG salt, water, trideceth -3 and 2-ethyl-1,3 hexanediol to Part I/Part II mixture in order given and mix with a Lightnin' mixer for 10 minutes.
2. Add a clear solution composed of EDTA in the remaining formula amount of water and mix until the desired viscosity is obtained.

For further stability improvement, pass the product through a Versator (deaeration).

## EXAMPLE I

A composition was prepared having the following formula:

6

| Ingredient | Percent by Wt. |
|---|---|
| Dimethyldioctadecyl ammonium hectorite (Bentone 38; NL Industries) | 1.00 |
| Cyclomethicone D-5 Pentamer (Union Carbide) | 21.18 |
| Propylene carbonate | 0.50 |
| Cyclomethicone D-4 Tetramer (Union Carbide) | 12.56 |
| Diprcpylene glycol | 0.04 |
| Dimethicone (350 cs.) (Silicone SF 18 (350); G.E.) | 10.00 |
| Colloidal silicon dioxide M-5 (Cab-O-Sil M-5, Cabot) | 0.20 |
| Polyethylene B-6, 6 micron (Polymist B-6; allied Chemical) | 5.00 |
| Ethylene Brassylate (Must T; Takasago) | 0.02 |
| Aluminum chlorohydrate (50% aqueous) | 48.00 |
| Trideceth-3 (Siponic TD-3; Alcolac) | 0.40 |
| 2-Ethyl-1,3 hexanediol (Amerchol Division of Union Carbide) | 0.80 |
| Distilled water | 0.30 |
| | 100.00 |

The composition was prepared by dispersing Bentone 38 in 13.0% of Cyclomethicone D-5 Pentamer and Lightnin' mixing until homogeneous. The propylene carbonate was added and the batch Lightnin' mixed until homogeneous. Mixing was then completed in a Waring Blender for 6 minutes using the low speed setting. The remaining Cyclomethicone D-5 Pentamer, Cyclomethicone D-4 Tetramer, dipropylene glycol, and Dimethicone were added and the batch was Lightnin' mixed until homogeneous. The colloidal silicon dioxide was added and the batch was Lightnin' mixed until homogeneous. The Polyethylene B-6 was slowly added and then the batch was Lightnin' mixed for 10 minutes. Mixing was completed in a Waring Blender for 6 minutes at low speed setting. The batch was allowed to cool to 110° F and Lightnin' mixing was continued. A uniform mixture of water, aluminum chlorohydrate, Trideceth-3 and 2-ethyl-1, 3 hexanediol were added and Lightnin' mixing was continued for ten minutes. Waring Blender mixing was continued until the desired viscosity was obtained. The finished product was then transferred to a storage container and packaged.

The finished product has a desired viscosity in the range of from about 500 cps to about 2500 cps and preferably will have a viscosity of from about 1000 to about 2000 cps.

EXAMPLE II

A composition was prepared having the following formula:

| Ingredients | Percent by Weight |
|---|---|
| Dimethyldioctadecyl ammonium hectorite (Bentone 38; NL Industries) | 1.20 |
| Cyclomethicone D-5 Pentamer (Union Carbide) | 22.64 |
| Propylene carbonate (Texaco Chemicals) | 0.50 |
| Cyclomethicone D-4 Tetramer (Union Carbide) | 15.10 |
| Dipropylene glycol | 0.04 |
| Dimethicone (350 cs.) (Silicone SF18 (350); G.E.) | 10.0 |
| Colloidal silicon dioxide M-5 | 0.20 |
| Polyethylene B-6, 6 Micron (Polymist B-6; Allied Chemical) | 5.00 |
| Ethylene Brassylate (Takasago; Musk T) | 0.02 |
| Butylated hydroxytoluene | 0.05 |
| Aluminum Zirconium Tetrachlorohydrex-Gly | 40.00 |
| 50% aqueous solution, W-374 (Wickenol 374; Wickhen Products) | |
| Trideceth-3 (Siponic TD-3; Alcolac) | 0.40 |
| 2-Ethyl-1.3-hexanediol (Amerchol Div. of Union Carbide) | 0.80 |
| Deionized water | 4.00 |
| Disodium edetate | 0.05 |
| | 100.00 |

The composition was prepared by dispersing Bentone 38 in 13% of the Cyclomethicone D-5 Pentamer, with Lightnin' mixer agitation, and mixed until homogeneous. The Propylene Carbonate was added and mixed until homogeneous. Mixing of the batch continued in a Waring Blender for 6 minutes using a low speed setting. The remaining 9.64% of the Cyclomethicone D-5 Pentamer, Cyclomethicone D-4 Tetramer, dipropylene glycol and Dimethicone SF 18 (350 cs.) were added and Lightnin' mixed for 5 minutes. The colloidal silicon dioxide M-5 was added and the batch was mixed for 5 minutes with a Lightnin' mixer. The Polyethylene B-6 was slowly added and the batch was mixed until homogenous. The butylated hydroxytoluene was added and the batch was Lightnin' mixed for 5 minutes. Mixing of the batch was continued for 6 minutes in a Waring Blender at a low speed setting. The batch was allowed to cool to 110° F while Lightnin' mixing. The Ethylene Brassylate was added and the batch was mixed with a Lightnin' mixer until homogenous. A uniform mixture of Aluminum Zirconium Tetrachlorohydrex-Gly, Trideceth-3, and 2 Ethyl-1,3 hexanediol was added and the batch was Lightnin' mixed for 10 minutes. A clear solution of water and disodium edetate was added and the batch was Lightnin' mixed for 10 minutes. Waring Blender mixing was continued until the desired viscosity was obtained.

The finished product has a desired viscosity in the range of 500 cps to 2500 cps and preferably 1000-2000 cps.

The finished product was then transferred to a storage container and packaged.

While the invention has been described with respect to various specific examples and embodiments, it is to be understood that the invention is not limited thereto and that it can be variously practiced within the scope of the following claims.

## Claims

1. In an antiperspirant suspension composition comprising an active antiperspirant ingredient, a volatile silicone ingredient, a non-volatile silicone ingredient and a suspending agent, the improvement comprising adding a sufficient amount of water to provide a wetness signal without rendering the composition unstable.

2. A composition according to claim 1 wherein the active antiperspirant ingredient is selected from the group consisting of aluminum salts and aluminum zirconium salts.

3. A composition according to claim 2 wherein the aluminum salt is selected from the group consisting of aluminum chlorohydrate, aluminum sesquichlorohydrate and aluminum dichlorohydrate.

4. A composition according to claim 1 wherein the active antiperspirant ingredient is aluminum chlorohydrate.

5. A composition according to claim 2 wherein the aluminum zirconium salt is selected from the group consisting of aluminum zirconium tetrachlorohydrex-Gly, aluminum zirconium pentachlorohydrex-Gly, and aluminum zirconium octachlorohydrex-Gly.

6. A composition according to claim 1 wherein the active antiperspirant ingredient is aluminum zirconium tetrachlorohydrex-Gly.

7. A composition according to claim 1 wherein the water is present in an amount from about 15 to about 30 weight percent based upon the total weight of the composition.

8. A composition according to claim 1 wherein the water is present in an amount from about 20 to about 25 weight percent based upon the total weight of the composition.

9. A composition according to claim 1 wherein the volatile silicone ingredient is cyclomethicone.

10. A composition according to claim 1 wherein the non-volatile silicone ingredient is dimethicone.

11. A hybrid roll-on antiperspirant composition having the following formulation, wherein the percentages stated are by weight:

| | |
|---|---|
| Dimethyldioctadecyl ammonium hectorite | 1.0 - 1.5% |
| Cyclomethicone (Pentamer and Tetramer) | qs. to 100% |
| Propylene carbonate | 0.30 - 0.80% |
| Dipropylene glycol | 0.04% |
| Dimethicone | 10.00% |
| Colloidal silicon dioxide | 0.1 - 0.5% |
| Polyethylene powder | 5.0% |
| Nonionic emulsifier (HLB 7) | 0.2 - 1.0% |
| Glycols (Propylene glycol, 2-Ethyl-1,3-hexanediol) | 0.8 - 2.0% |
| Water | 20 - 24% |
| Aluminum or aluminum zirconium antiperspirant salt | 18 - 24% |